# EUROPEAN PATENT SPECIFICATION

(11) **EP 4 110 343 B1**
(45) Date of publication and mention of the grant of the patent: **08.04.2026**
(21) Application number: 21709501.7
(22) Date of filing: 19.02.2021
(51) Int. Cl.: A61K 31/519, A61P 19/00, A61P 19/10, A61P 25/00, A61P 25/28

(54) **4-PYRROLIDIN-1-YL-5-P-TOLYL-THIENO[2,3-D] PYRIMIDINE FOR USE IN THE TREATMENT OF AGING-ASSOCIATED AND PREMATURE AGING DISEASES THROUGH RESTORED CHROMOSOMAL STABILITY AND INHIBITION OF CELLULAR SENESCENCE**
4-PYRROLIDIN-1-YL-5-P-TOLYL-THIENO[2,3-D]-PYRIMIDIN ZUR VERWENDUNG BEI DER BEHANDLUNG VON ALTERUNGS-ASSOZIIERTEN UND VORZEITIGEN ALTERUNGSKRANKHEITEN DURCH WIEDERHERSTELLUNG DER CHROMOSOMALEN STABILITÄT UND HEMMUNG DER ZELLULÄREN SENESZENZ
4-PYRROLIDIN-1-YL-5-P-TOLYL-THIÉNO[2,3-D] PYRIMIDINE DESTINÉE À ÊTRE UTILISÉE DANS LE TRAITEMENT DE MALADIES ASSOCIÉES AU VIEILLISSEMENT ET AU VIEILLISSEMENT PRÉMATURÉ PAR LA STABILITÉ CHROMOSOMIQUE RESTAURÉE ET L'INHIBITION DE LA SÉNESCENCE CELLULAIRE

(30) Priority: 19.02.2020 PT 2020116123
(43) Date of publication of application: 04.01.2023
(73) Proprietor: Instituto De Biologia Molecular E Celular - IBMC, 4200-135 Porto (PT)
(72) Inventor: CARVALHO LOGARINHO SANTOS, Elsa Clara, 4200-135 Porto (PT)
(74) Representative: Ferreira Pinto, Francisca
(86) International application number: PCT/PT2021/050004
(87) International publication number: WO 2021/167478

(56) References cited:
- EP-A1- 3 511 005
- WO-A1-2017/002120
- ANNE-LAURE EGESIPE ET AL: "Metformin decreases progerin expression and alleviates pathological defects of Hutchinson-Gilford progeria syndrome cells", NPJ AGING AND MECHANISMS OF DISEASE, vol. 2, no. 1, 10 November 2016 (2016-11-10), XP055484935, DOI: 10.1038/npjamd.2016.26
- RYAN W. DELLINGER ET AL: "Repeat dose NRPT (nicotinamide riboside and pterostilbene) increases NAD+ levels in humans safely and sustainably: a randomized, double-blind, placebo-controlled study", NPJ AGING AND MECHANISMS OF DISEASE, vol. 3, no. 1, 24 November 2017 (2017-11-24), XP055482304, DOI: 10.1038/s41514-017-0016-9
- "Cytometry: Part B", vol. 158, 1 January 2020, SAN DIEGO [U.A.] : ACAD. PRESS, US, article WARREN JESSICA D. ET AL: "A comparative analysis of methods to measure kinetochore-microtubule attachment stability", pages: 91 - 116, XP055795655, DOI: 10.1016/bs.mcb.2020.01.004
- MAHMOUDI SALAH ET AL: "Turning back time with emerging rejuvenation strategies", NATURE CELL BIOLOGY, MACMILLAN MAGAZINES LTD, GB, vol. 21, no. 1, 2 January 2019 (2019-01-02), pages 32 - 43, XP036664258, ISSN: 1465-7392, [retrieved on 20190102], DOI: 10.1038/S41556-018-0206-0
- SOTO-GAMEZ ABEL ET AL: "Therapeutic interventions for aging: the case of cellular senescence", DRUG DISCOVERY TODAY, ELSEVIER, AMSTERDAM, NL, vol. 22, no. 5, 19 January 2017 (2017-01-19), pages 786 - 795, XP085018036, ISSN: 1359-6446, DOI: 10.1016/J.DRUDIS.2017.01.004
- YI ZHU ET AL: "The Achilles' heel of senescent cells: from transcriptome to senolytic drugs", AGING CELL, vol. 14, no. 4, 1 August 2015 (2015-08-01), GB, pages 644 - 658, XP055342891, ISSN: 1474-9718, DOI: 10.1111/acel.12344
- LY D H ET AL: "Mitotic misregulation and human aging", SCIENCE, AMERICAN ASSOCIATION FOR THE ADVANCEMENT OF SCIENCE, US, vol. 287, no. 5462, 31 March 2000 (2000-03-31), pages 2486 - 2492, XP002242889, ISSN: 0036-8075, DOI: 10.1126/SCIENCE.287.5462.2486
- BARROSO-VILARES MONIKA ET AL: "Small-molecule inhibition of aging-associated chromosomal instability delays cellular senescence", EMBO REPORTS, vol. 21, no. 5, 6 May 2020 (2020-05-06), GB, XP055794722, ISSN: 1469-221X, DOI: 10.15252/embr.201949248
- TOVINI LAURA ET AL: "Towards restoring proper chromosome segregation and preventing ageing", EMBO REPORTS, vol. 21, no. 5, 28 April 2020 (2020-04-28), GB, XP055795656, ISSN: 1469-221X, Retrieved from the Internet <URL:https://onlinelibrary.wiley.com/doi/full-xml/10.15252/embr.202050322> DOI: 10.15252/embr.202050322

## Description

### Technical field of the invention

The present invention relates to the technical field of human health; medical science; preparations for medical purposes; in particular, medicinal preparations containing organic active ingredients, namely heterocycles, specifically those which contain the thienopyrimidine moiety.

Thiophene containing compounds are well known to exhibit various biological effects. Heterocycles containing the thienopyrimidine moiety Thiophene containing compounds are well known to exhibit various biological effects. Heterocycles containing the thienopyrimidine moiety

### State of the art

Aging is characterized by the progressive disruption of key biological processes and correlates with the extensive accumulation of macromolecular damage over time. As a consequence, tissue and organ homeostasis is perturbed, which contributes to an overall deterioration of physiological functions. Potential drivers of the aging process have been identified and categorized into hallmarks. Molecular hallmarks comprise DNA damage, telomere attrition, epigenetic remodeling, loss of proteostasis, and mitochondrial dysfunction (Lopez-Otin, Blasco et al., 2013). Cellular and organismal features of aging include cellular senescence, deregulated nutrient sensing, and stem cell exhaustion (Lopez-Otin et al., 2013). In recent years, several rejuvenation strategies emerged that target these hallmarks (Mahmoudi, Xu et al., 2019). Amongst them, metabolic manipulations and senescent cell ablation (or senolysis) have become popular. Senescent cells, which undergo a permanent cell cycle arrest in response to stressors and exhibit stereotyped phenotypic changes, have been shown to contribute to aging (Childs, Durik et al., 2015, van Deursen, 2014). Their targeted clearance was evidenced to attenuate or even prevent age-associated conditions (Abdul-Aziz, Sun et al., 2019, Bussian, Aziz et al., 2018, Childs, Baker et al., 2016, Farr, Xu et al., 2017, Jeon, Kim et al., 2017, Roos, Zhang et al., 2016) and to improve lifespan of naturally aged wild-type mice (Baker, Childs et al., 2016). Senolysis also extended healthspan in progeroid mice that experience chromosomal instability (CIN) as a result of mitotic checkpoint signaling defects (Baker, Wijshake et al., 2011). This suggested a possible link between CIN and aging through the accrual of senescent cells. More recently, this correlation was further supported by studies showing that loss of chromosome segregation fidelity in otherwise karyotypically stable human cells prompts a CIN-driven senescence signature accompanied by the senescence-associated secretory phenotype (or SASP) (He, Au et al., 2018, Santaguida, Richardson et al., 2017). Thus aneuploidy, a state of abnormal chromosome number for long reported to occur with age (Iourov, Vorsanova et al., 2009, Mosch, Morawski et al., 2007, Mukherjee, Alejandro et al., 1996, Mukherjee & Thomas, 1997, Nagaoka, Hassold et al., 2012, Stone & Sandberg, 1995), may significantly contribute to the aging process.

Maintenance of chromosomal stability is ensured through the tightly controlled and timely organization of microtubules (MTs) into a bipolar mitotic spindle and microtubule attachment to the complex proteinaceous structures (kinetochores) at the centromeres of all chromosomes prior to their segregation toward opposite poles. Defects in the spindle assembly checkpoint (SAC) that prevents anaphase onset in the presence of unattached kinetochores, as well as the premature separation of sister chromatids due to cohesion defects, will give rise to aneuploid daughter cells (Compton, 2011). In addition, a major mechanism generating aneuploidy is the persistence of erroneous merotelic kinetochore-microtubule (k-MT) attachments, in which a single kinetochore bound to MTs from opposite poles is left uncorrected, generating an anaphase lagging chromosome and a micronuclei(MN) in telophase (Cimini & Degrassi, 2005). As increases in aneuploidy have been observed with aging, there is the possibility that the mechanisms required to maintain chromosomal stability might deteriorate with age (Macedo, Vaz et al., 2017). Although work on cells and mice with CIN have pointed to a link between chromosomal abnormalities and aging, the mitotic behavior of naturally aged cells was only recently characterized. Analysis of primary human dermal fibroblasts derived from neonatal to octogenarian individuals revealed a progressive loss of proliferative capacity and mitotic dysfunction with age. As a result of the global mitotic gene shutdown caused by the repression of the transcription factor Forkhead box M1 (FoxM1), elderly cells experience chromosome segregation defects that were found to ultimately trigger a full senescence phenotype (Macedo, Vaz et al., 2018). Altogether this raises the problem that loss of mitotic fidelity with aging underlies mild CIN, thus favoring the accrual of aneuploid senescent cells. Uncovering the yet unknown therapeutics to prevent the mechanism(s) by which aging triggers chromosome segregation defects, and resulting aneuploidy, is paramount for treating aging-associated (atherosclerosis, cardiovascular disease, cancer, arthritis, cataracts, osteoporosis, type 2 diabetes, hypertension and Alzheimer's disease) and premature aging diseases (progeroid syndromes such as Bloom Syndrome; Cockayne Syndrome Type I -216400 or Type III - 216411; Hutchinson-Gilford Progeria Syndrome; Mandibuloacral Dysplasia with Type A Lipodystrophy; Progeria of Adult Onset; Progeroid Syndrome, Neonatal Rothmund-Thomson Syndrome; Seip Syndrome and Werner Syndrome) in light of all recent findings connecting CIN, senescence and aging.

Soto-Gamez Abel et al: "Therapeutic interventions for aging: the case of cellular senescence", DRUG DISCOVERY TODAY, ELSEVIER, AMSTERDAM, NL, vol.22, no.5, 19 January 2017 (2017-01-19), pages 786-195, XP085018036, ISSN: 1359-6446, DOI: 10.1016/J.DRUDIS. 2017.01.004 **refers to already known treatments of senescence and related disease or disorders with active ingredients not related to the present subject matter.**

YI ZHU et al: "The Achilles' heel of senescent calls: from transcriptome to senolytic drugs", AGING CELL, vol 14, no.4, 1 August 2015 (2015-08-01), pages 644-658, XP055342891, GB ISSN; 1474-9718, DOI: 10.1111/acel.12344 **discloses treatment of senescence with dasatinib and quercetin.**

WO 2017/002120 **discloses compounds under a general formula not related to the present invention for use in the selective elimination of senescent cells.**

EP3511005 **discloses an inhibitor of JAK-STAT pathway for use in treating Hutchinson-Gilford progeria syndrome.**

LY D H eta al: "Mitotic misregulation and human aging", SCIENCE, AMERICAN ASSOCIATION FOR THE ADVANCEMENT OF SCIENCE, US, vol.287, no. 5462, 31 March 2000 (31-03-2000, pages 2486-2492, XP002242889, ISSN: 0036-8075, DOI: 10.1126/SCIENCE.287.5462.2486 **studies the role of mitotic misregulation in human ageing and explores the possible relationship between MCAK and chromosome lagging.**

### Summary of the Invention

In the present invention we disclose that human dermal fibroblasts derived from elderly individuals have lower levels of proteins required for establishment of proper kinetochore-microtubule (k-MT) attachments, including MT-destabilizing kinesins involved in the correction of merotelic k-MT interactions. As a result of compromised error correction, improper k-MT attachments persist into anaphase giving rise to aneuploid daughter cells. Notably, pharmacological rescue of MT-destabilizing kinesin-13 activity reestablished chromosome segregation accuracy in elderly cells, concomitantly with a reduction in cellular senescence. Consequently, strategic destabilization of k-MT attachments may be a potential therapeutic strategy to counteract age-associated senescence and thereby act to improve healthspan and treat premature aging diseases.

As such, the present invention refers to the subject matter as defined in claims 1 to 8: 4-Pyrrolidin-1-Yl-5-P-Tolyl-Thieno[2,3-D] Pyrimidine for use in the treatment of aging-associated diseases, such as atherosclerosis, cardiovascular disease, arthritis, cataracts, osteoporosis, type 2 diabetes, hypertension and Alzheimer's disease.

In another embodiment of the present invention, the previous substances for use in the treatment of aging-associated diseases are comprised in a pharmaceutical composition of a medicament.

In another embodiment, the previously mentioned substance and compositions for use in the treatment of aging-associated diseases are administered systemically.

In another embodiment, the said substance and compositions for use in the treatment of aging-associated diseases are administered orally.

In another embodiment, the said substance and compositions for use in the treatment of aging-associated diseases are administered by local injection.

The herein disclosed invention also refers to 4-Pyrrolidin-1-Yl-5-P-Tolyl-Thieno[2,3-D]Pyrimidine for use in the treatment of premature aging diseases, such as Bloom Syndrome; Cockayne Syndrome Type I -216400 or Type III - 216411; Hutchinson-Gilford Progeria Syndrome; Mandibuloacral Dysplasia with Type A Lipodystrophy; Progeria of Adult Onset; Progeroid Syndrome, Neonatal Rothmund-Thomson Syndrome; Seip Syndrome and Werner Syndrome.

In another embodiment of the present invention, the previous substances for use in the treatment of premature aging diseases are comprised in a pharmaceutical composition of a medicament.

In another embodiment, the previously mentioned substance and compositions for use in the treatment of premature aging diseases are administered systemically.

In another embodiment, the said substance and compositions for use in the treatment of premature aging diseases are administered orally.

In another embodiment, the said substance and compositions for use in the treatment of premature aging diseases are administered by local injection.

### Detailed description of the Invention

The invention stems from the discovery that defective microtubule dynamics occurs in elderly mitotic cells compromising biological processes that rely in microtubule dynamics, such as genomic stability.

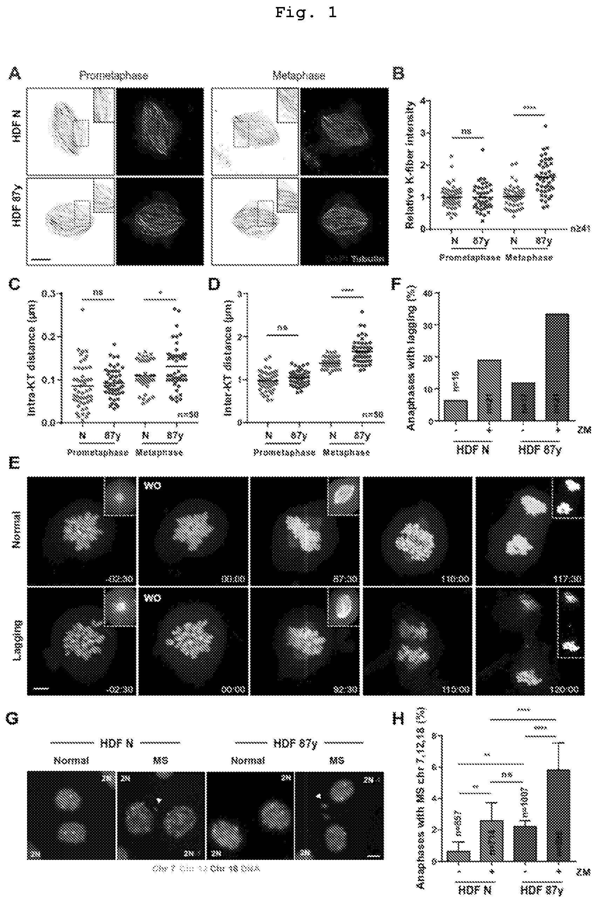

In one embodiment, by comparing kinetochore fibers (k-fiber) in human dermal fibroblasts (HDFs) derived from young and elderly healthy Caucasian males it is disclosed how changes in k-MT attachment stability underlie the mild chromosomal instability (CIN) observed with age. In this embodiment, the calcium-induced depolymerization of non-kinetochore microtubules reveals that elderly cells have increased k-fiber intensity levels at the metaphase stage when compared to neonatal cells (Fig 1A,B). Intra- and inter-kinetochore distances of aligned chromosomes in elderly metaphase cells are also increased (Fig 1C,D). Taken together, these data indicate that elderly cells have an increased number of k-MT attachments in metaphase. Furthermore, MT occupancy at kinetochores is higher in elderly cells and consequently an increased number of erroneous k-MT attachments is observed.

Through assessment of the efficiency of error correction using the reversible inhibition of kinesin-5 with S-trityl-L-cysteine (STLC) to induce transient monopolar spindles and potentiate the formation of erroneous attachments and through live cell imaging of cells expressing H2B-GFP/α-Tubulin-mCherry its disclosed that elderly cells are two times more likely to exhibit lagging chromosomes following STLC washout than their young counterparts (11.8% vs. 6.3%) (Fig 1E,F). Fluorescence in situ hybridization (FISH) analysis for 3 chromosome pairs showed that chromosome mis-segregation is higher in elderly dividing cells (2.22% vs. 0.63%) (Fig 1G,H).

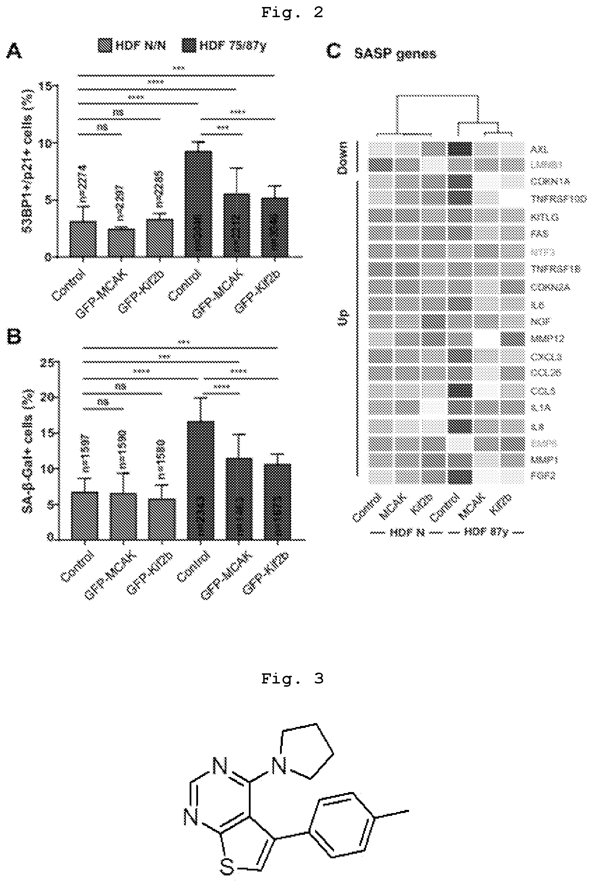

In another embodiment of the present invention its disclosed how strategic destabilization of k-MTs delays senescence, how overexpression of kinesin-13 proteins MCAK and Kif2b can have implications on senescence and how counteracting age-associated mild CIN can delay the development of full senescence in elderly cells. In this embodiment, as an implication of kinesin-13 overexpression, the percentage of cells exhibiting senescence biomarkers was reduced upon improved error correction efficiency (Fig 2A,B). Furthermore, from a custom list of senescence-related genes (Fig 2C), differential expression between young and elderly cells according to the expected was observed for 20 genes, in which 17 were correctly altered following overexpression of kinesin-13 proteins. Taken together this embodiment discloses how kinesin-13 proteins overexpression in aged cells restores chromosome segregation fidelity. This in turn has a positive impact on the elderly cell population, as significant improvements in the senescence-associated transcriptome signature match with delayed emergence of fully senescent cells permanently arrested in the cell cycle.

In another embodiment of the present invention, its disclosed how small molecule inhibition of age-associated chromosome mis-segregation delays senescence. In this embodiment, we disclose new medical uses of a small molecule agonist that specifically potentiates the activity of the kinesin-13 protein MCAK, with the general chemical formula: 4-Pyrrolidin-1-yl-5-p-tolyl-thieno[2,3-d] pyrimidine and termed UMK57 (Fig 3).

4-Pyrrolidin-1-Yl-5-P-Tolyl-Thieno[2,3-D] Pyrimidine (UMK57) can be obtained commercially from AOBIOUS, INVIVOCHEM and MREDKOO Biosciences.

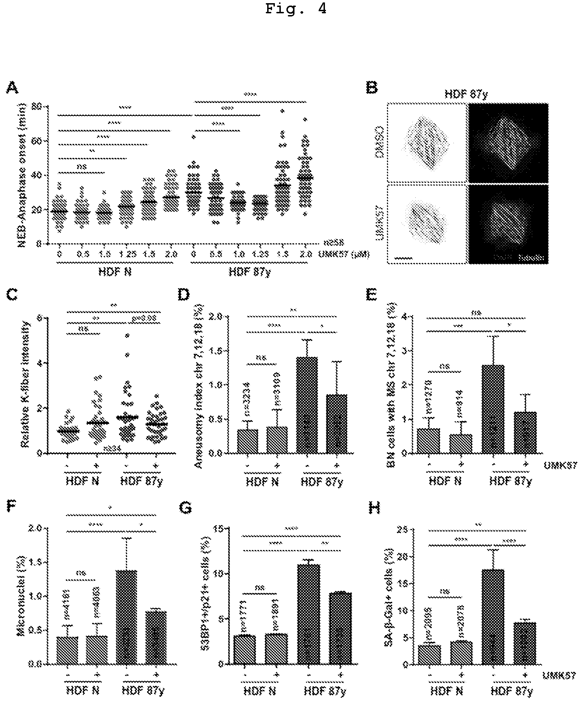

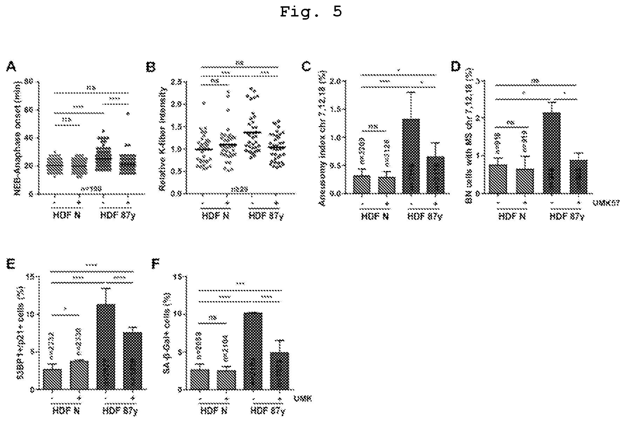

In this embodiment its disclosed how surprisingly this agonist provides a small molecule approach, as an alternative to genetic overexpression, to suppress ageing-related CIN. Through following cells exposed to increasing concentrations of UMK57 under 24-hour long-term time-lapse microscopy its disclosed that 1µM is sufficient to rescue the increased mitotic duration in elderly cells, while having no noticeable effect on the mitotic progression of neonatal cultures (Fig 4A). In agreement with the rescued mitotic delay, calcium-stable k-fiber intensity analysis in UMK57-treated elderly cells revealed that enhanced MCAK activity decreases the number of stable k-MT attachments in metaphase (Fig 4B, C). FISH analysis of 3 chromosome pairs in both interphase cells and cytokinesis-blocked binucleated (BN) cells showed that UMK57 decreases the levels of aneuploidy and the chromosome mis-segregation rate in elderly cell populations (Fig 4D,E). Also, MN levels were scored and found to be partly decreased in elderly cells upon 24-hour exposure to the MCAK agonist (Fig 4F). These embodiments indicate that age-associated mild CIN can be rescued using the said small molecule agonist of the kinesin-13 protein MCAK. Furthermore UMK57-induced MCAK activity could delay cellular senescence. A 24-hour treatment is sufficient to partially rescue the percentage of cells exhibiting the senescence biomarkers 53bp1+p21 and SA-β-galactosidase activity (Fig 4G,H). Taken together, these embodiments show how strategic destabilization of k-MT attachments aids in the correction of improper k-MT attachments, while acting to counteract cellular senescence with aging. Additionally, the beneficial effect of UMK57 persists over long-term exposure. After 96-hour treatment, the mitotic delay of elderly cells is rescued (Fig 5A). In agreement, decreased k-fiber intensity levels in metaphase are still observed after 96 hours (Fig 5B). FISH analyses on interphase and BN cells shows that aneuploidy (Fig 5C) and chromosome mis-segregation (Fig 5D) are also inhibited after 96 hours. Furthermore, we disclose a long-term repression of cellular senescence, demonstrated by the partial rescue in senescence markers (Fig 5E,F).

In summary, impaired kinesin-13 activity can be established as a mechanistic link between chromosome mis-segregation and senescence in naturally aged cells and the herein disclosed embodiments increasing k-MT detachment rate, through the overexpression of kinesin-13 proteins or through treatment with UMK57, rescue **k-**fiber intensity levels, improve error correction and the segregation fidelity in the aged fibroblasts. The disclosed embodiments also reveal that modulation of kinesin-13 activity inhibits the accrual of cells exhibiting senescence biomarkers and notably, small molecule modulation of age-associated CIN significantly delays senescence.

Even though modulation of CIN solely acts on mitotically active aged cell/tissue populations, there are substantial arguments for it to be taken into consideration as an anti-aging strategy. First, different types of proliferative cells support the function of stem and differentiated cell pools via paracrine signaling, by secreting bioactive molecules. Thus, delaying the emergence of full senescence in proliferative cell types as a result of improved chromosome segregation fidelity likely counteracts microenvironmental changes in aged tissues. Second, modulation of CIN encompasses several advantages of senolysis since it prevents the generation of fully senescent cells and their detrimental paracrine signaling. Furthermore, emergent rejuvenation strategies such as dietary regimens, cellular reprogramming and senolysis, primarily target cells with proliferative potential/capacity (adult stem cells, vascular and connective tissue cells) or with loss of proliferative capacity (senescent cells). Consequently, safeguarding cell proliferation with fidelity should delay the disruption of tissue homeostasis with age.

The small molecule modulation of kinesin-13 MCAK acts upstream in the order of events, by reestablishing mitotic competence and diluting out senescent cells. As senescence is a strong contributor to the aging process another embodiment of the present invention refers to 4-Pyrrolidin-1-Yl-5-P-Tolyl-Thieno[2,3-D] Pyrimidine for use in the treatment of aging-associated diseases, such as atherosclerosis, cardiovascular disease, arthritis, cataracts, osteoporosis, type 2 diabetes, hypertension and Alzheimer's disease.

In another embodiment of the present invention, the previously mentioned substances for use in the treatment of aging-associated diseases are comprised in a pharmaceutical composition of a medicament.

UMK57 pharmacological rescue of MCAK activity is a means to delay cellular aging. This supports another embodiment of the present invention that refers to 4-Pyrrolidin-1-yl-5-p-tolyl-thieno[2,3-d]pyrimidine, for use in the treatment of Premature Aging Diseases and Syndromes, such as: Bloom Syndrome; Cockayne Syndrome Type I -or Type III; Hutchinson-Gilford Progeria Syndrome; Mandibuloacral Dysplasia with Type A Lipodystrophy; Progeria of Adult Onset; Progeroid Syndrome, NeonatalRothmund-Thomson Syndrome; Seip Syndrome; Werner Syndrome and other of the same type.

In another embodiment of the present invention, the previous substances for use in the treatment of premature aging diseases are comprised in a pharmaceutical composition of a medicament.

### Other Examples

In another embodiment, the previously mentioned substance and compositions for use in the treatment of aging-associated and premature aging diseases are administered systemically.

In another embodiment, the said substance and compositions for use in the treatment of aging-associated and premature aging diseases are administered orally.

In another embodiment, the said substance and compositions for use in the treatment of aging-associated and premature aging diseases are administered by local injection.

### Brief description of the Figures

**Figure 1****: Impaired k-MT error correction with advancing age.** (A) Representative images and (B) quantification of calcium-stable k-fiber intensity levels by immunofluorescence analysis of n≥41 tubulin-stained mitotic cells of neonatal (N) and elderly (87y) human dermal fibroblasts (HDF) at prometaphase and metaphase stages. Intensity levels were normalized to neonatal samples. Scale bar, 5µm. (C) Intra-kinetochore distance (between Hec1 and ACA immunostainings of a kinetochore; n=50) in elderly vs. neonatal cells in prometaphase and metaphase. (D) Inter-kinetochore distance (between Hec1 staining of sister kinetochores; n=50) in elderly vs. neonatal cells in prometaphase and metaphase. (E) Live cell imaging of neonatal (N) and elderly (87y) fibroblasts expressing H2B-GFP/α-Tubulin-mCherry treated with kinesin-5 inhibitor (STLC) to induce monopolar spindles, followed by washout (WO) into medium with DMSO or ZM447439 (500nM). Representative movie frame series of dividing cells that underwent correct (Normal) and incorrect (Lagging) chromosome segregation are shown. Time, min:sec. Scale bar, 5µm. (F) Quantification of anaphases with lagging chromosomes in n=cells scored by confocal microscopy following WO into medium with DMSO (-) or ZM447439 (+). (G) Representative images of anaphases without (top) and with (bottom) mis-segregation (MS), FISH-stained for three chromosome pairs (7, 12 and 18). Scale bar, 10µm. (H) Percentage of anaphases with MS in neonatal (N) vs. elderly (87y) n=cells scored by FISH analysis. Values shown are mean ± s.d. of at least two independent experiments. ns p>0.05, * p<0.05, ** p<0.01, and **** p<0.0001 by two tailed (B-D) Mann-Whitney test and (H) chi-square test. Human dermal fibroblasts (HDFs) retrieved from skin samples of neonatal (No. GM21811, Coriell Institute; No. DFM021711A, Zen Bio) and octogenarian (No. AG07135; AG13993; AG09271; AG10884; all from Coriell Institute) Caucasian males were used. All donors were reported as "healthy". For all experiments, HDFs were seeded at 1x104 cells per cm2 of growth area in minimal essential medium Eagle-Earle (MEM) supplemented with 15% fetal bovine serum (FBS), 2mM L-glutamine, and 1x antibiotic-antimycotic (all from Gibco, Thermo Fisher Scientific). Only early passage dividing fibroblasts (up to passage 3-5) with cumulative population doubling level PDL < 24 were used.
   For calcium-stable k-fiber analysis fibroblasts grown on sterilized glass coverslips coated with 50µg/ml fibronectin (F1141, Sigma-Aldrich) were incubated in Calcium buffer (100mM PIPES, 1mM MgCl2, 1mM CaCl2, 0.5% Triton X100, pH=6.8) for 5 min and fixed with 4% paraformaldehyde/0.25% glutaraldehyde in PBS for 15 min, both at 37°C. Next, cells were rinsed first in PBS, then in TBS (50mM Tris-HCl, pH=7.4, 150mM NaCl), and permeabilized in TBS + 0.3% Triton-X100 for 7 min. Blocking was performed with 10% FBS + TBS + 0.05% Tween-20 for 1 hr and cells were then incubated with mouse anti-α-tubulin (T5168, Sigma-Aldrich) antibody diluted at 1:1500 in 10% FBS + TBS + 0.05% Tween-20. The secondary antibodies AlexaFluor-488 and -568 (Life Technologies) were used at 1:1500 in 5% FBS + TBS + 0.05% Tween-20. DNA was counterstained with 0.5µg/ml DAPI (Sigma-Aldrich) and coverslips mounted on slides.
   Proteasome inhibitor MG-132 (474790, EMD Millipore) was used at 5µM for 2 hrs to arrest cells at the metaphase stage. Cytochalasin D (C8273, Sigma-Aldrich) was used at 1µM for 24 hrs to block cytokinesis. Fibroblasts were treated with 2.5µM STLC (2191, TOCRIS) for 5 hrs to inhibit kinesin-5 activity and induce monopolar spindles, followed by a washout into fresh medium with 500nM of Aurora kinase B inhibitor ZM447439 (S1103, Selleckchem) to potentiate chromosome segregation errors. To enrich the Mitotic Index for mitotic cell shake-off, STLC was used at 5µM during 16 hrs.
   For immunofluorescence, fibroblasts were grown on sterilized glass coverslips coated with 50µg/ml fibronectin (F1141, Sigma-Aldrich) and fixed with 4% paraformaldehyde in PBS for 20min. Following fixation, cells were rinsed in PBS, permeabilized in PBS + 0.3% Triton-X100 for 7 min and then blocked in 10% FBS + PBS for 1 hr. Both, primary and secondary antibodies were diluted in PBS + 0.05% Tween-20 containing 5% FBS as follows. Primary antibodies: rabbit anti-53BP1 (4937, Cell Signaling Technology), 1:100; mouse anti-p21 (SC-6246, Santa Cruz Biotechnology), 1:800; mouse anti-Aurora B (Aim-1; 611082, BD Biosciences), 1:500; rabbit anti-cGAS (15102, Cell Signaling Technology), 1:200; mouse anti-Hec1 (ab3613, Abcam), 1:1500; mouse anti-Plk1 (SC-17783, Santa Cruz Biotechnology), 1:2000; rabbit anti-MCAK (Manning, Ganem et al., 2007), 1:5000; mouse anti-α- tubulin (T5168, Sigma-Aldrich), 1:1500; human anti-centromere antibody (ACA; kindly provided by Dr. W. C. Earnshaw), 1:3000; rabbit anti-Aurora B phosphoT232 (600-401-677, ROCKLAND), 1:1000. Secondary antibodies: AlexaFluor-488, -568 and -647 (Life Technologies), all 1:1500. DNA was counterstained with 0.5µg/ml DAPI (Sigma-Aldrich) and coverslips mounted on slides with proper mounting solution.
   In fluorescence microscopy, cells with calcium-stabilized k-fibers or stained for specific kinetochore/centromere-bound proteins (Aurora B/Aim-1, Hec1/Ndc80, MCAK, Plk1, pAuroraB T232 and ACA) were imaged using a Zeiss AxioImager Z1 (Carl Zeiss, Oberkochen, Germany) motorized upright epifluorescence microscope, equipped with an Axiocam MR camera and operated by the Zeiss Axiovision v4.7 software. Z-stacks (0.24 µm) covering the entire volume of individual mitotic cells were collected using a PlanApo 63x/1.40 NA objective. Image deconvolution was performed with the AutoQuant X2 software (Media Cybernetics).
   For image analysis, both live-cell phenotypes (mitotic duration, lagging chromosomes) and fixed-cell experiments (protein intensity, k-fiber intensity, KT distances, FISH, MN counts, cGAS positivity and SA biomarkers) were blindly quantified using ImageJ/Fiji software. For the analysis of protein intensity levels, the kinetochore area was taken into consideration and Aurora B/Aim-1, Hec1/Ndc80, MCAK, Plk1 and pAuroraB T232 levels were then corrected for the background and normalized to ACA levels (also corrected for the background). For analysis of calcium-stable k-fibers, α-tubulin intensity levels were normalized for the mitotic spindle area of each individual cell and background-corrected. For MN frequencies, interphase cells with DNA aggregates separate from the primary nucleus were considered, while interphase cells with an apoptotic appearance were excluded. DNA aggregates co-localizing with cGAS were scored as MN positive for cGAS. For the analysis of SA biomarkers (53bp1/p21 and SA-β-galactosidase), fluorescence intensity thresholds were set and used consistently for all samples within each experiment. In case of SA-β-galactosidase activity, only cells displaying >5 fluorescent granules were considered positive.
   In phase-contrast live cell imaging fibroblasts grown in ibiTreat polymer-coated µ-slide (Ibidi GmbH, Germany) were imaged using a Zeiss Axiovert 200M inverted microscope (Carl Zeiss, Oberkochen, Germany) equipped with a CoolSnap camera (Photometrics, Tucson, USA), XY motorized stage and NanoPiezo Z stage, under controlled temperature, atmosphere, and humidity. Neighbor fields (20-25) were imaged every 2.5 min for 24-48 hrs, using a 20x/0.3 NA Aplan objective. The "Stitch Grid" (Stephan Preibisch) plugin from ImageJ/Fiji software was used to stitch neighboring fields for image analysis.
   For spinning-disk confocal microscopy, fibroblasts were grown in ibiTreat polymer-coated 35mm µ-dishes (Ibidi GmbH, Germany) and imaged using the Andor Revolution XD spinning-disk confocal system (Andor Technology, Belfast, UK), equipped with an electron-multiplying charge-coupled device iXonEM Camera and a Yokogawa CSU 22 unit based on an Olympus IX81 inverted microscope (Olympus, Southend-on-Sea, UK). The system was driven by Andor IQ software and laser lines at 488 and 561 nm were used for excitation of GFP and mCherry, respectively. Z-stacks (0.8-1.0 µm) covering the entire volume of individual mitotic cells were collected every 1.5 min using a PlanApo 60x/1.4 NA objective. ImageJ/Fiji software was used to edit the movies in which every image represents a maximum-intensity projection of all z-planes.
   For FISH, MN counts, cGAS immunofluorescence and SA biomarkers, images were captured with the IN Cell Analyzer 2000 (GE Healthcare, UK) equipped with a Photometrics CoolSNAP K4 camera and driven by the GE IN Cell Analyzer 2000 v5.2 software, using a Nikon 20x/0.45 NA Plan Fluor objective and a Nikon 40x/0.95 NA Plan Fluor objective, respectively.
**Figure 2****: Overexpression of kinesin-13 proteins MCAK and Kif2b delays senescence in fibroblast cultures from elderly donors.**
   Neonatal (N/N) and elderly (75/87y) human dermal fibroblasts (HDF) transduced with empty, GFP-MCAK or GFP-Kif2b lentiviral plasmids were analyzed for senescence. (A) Percentage of n=cells staining positive for double immunostaining of Cdkn1a/p21 (cell cycle inhibitor) and 53BP1 (≥1 foci; DNA damage). (B) Percentage of n=cells staining positive (right) for SA-β-galactosidase activity (SA-β-gal). Heatmaps of differentially expressed (C) SASP and senescence genes. Gene symbols highlighted in grey indicate genes that were not modulated by overexpression of Kinesin-13 proteins. Z-score row color intensities indicate higher (red) to lower (blue) expression. Values are mean ± s.d. of at least two independent experiments. ns p>0.05, *** p<0.001, **** p<0.0001 by two tailed chi-square test.
   To assemble pLVX-Tight-Puro plasmids for lentiviral transduction and expression of GFP-MCAK and mEOS-α-Tubulin, BamHI-NotI-tailed fragments were PCR-amplified from GFP-MCAK (gift from Dr. Linda Wordeman) and mEos2-Tubulin-C-18 (#57432, Addgene), respectively. To generate pLVX-Tight-Puro-GFP-Kif2b, a NotI-MluI-tailed fragment was amplified from GFP-Kif2b (gift from Dr. Linda Wordeman). The PCR products were then ligated into the BamHI and NotI, or NotI and MluI restriction sites of digested pLVX-Tight-Puro vector (Clontech). All primers used for PCR amplifications are listed in Table S1.
   Lentiviruses were produced according to the Lenti-X Tet-ON Advanced Inducible Expression System (Clontech). HEK293T helper cells were transfected with packaging plasmids pMd2.G and psPAX2 using Lipofectamine 2000 (Life Technologies) to generate responsive lentiviruses carrying pLVX-Tight-Puro, pLVX-TightPuro-H2B-GFP/α-tubulin-mCherry (Macedo et al., 2018), pLVX-Tight-Puro-GFP-MCAK, pLVX-Tight-Puro-GFP-Kif2b or pLVX-Tight-Puro-mEOS-α-Tubulin, as well as transactivator lentiviruses carrying the rtTA expressing construct (pLVX-Tet-On Advanced). Human fibroblasts were then co-infected for 6 hrs with both the responsive and the transactivator lentiviruses (2:1 ratio) in the presence of 8ug/ml polybrene (AL-118, Sigma-Aldrich). Co-transduction was induced with 500ng/ml doxycycline (D9891, Sigma-Aldrich). Transfection efficiencies of all experiments were determined by scoring the number of fluorescent cells, or protein levels by western blot analysis.
   Subpopulations of GFP-positive cells were sorted by Fluorescence-activated cell sorting (FACS) to validate lentiviral transduction of pLVX-Tight-Puro-GFP-MCAK, pLVX-Tight-Puro-GFP-Kif2b and pLVX-Tight-Puro-EOS-α-Tubulin. FACS sorting was performed using a FACSAria^{™} I Cell Sorter (BD Biosciences), with the laser line of 488 nm. Dead cells and subcellular debris were excluded using gates based on forward scatter area (FSC-A) vs. side scatter area, while cell doublets and clumps were excluded through FSC-A vs. FSC-width plot. The signal was detected using the APC-A channel and gates designed based on the respective auto-fluorescent control.
   For SA-β-gal assay cells were incubated in culture medium containing 100nM Bafilomycin A1 (B1793, Sigma-Aldrich) for 90 min to induce lysosomal alkalization. The fluorogenic substrate for β-galactosidase, fluorescein di-β-D-galactopyranoside (33µM; F2756, Sigma-Aldrich) or DDAO galactoside (10µM; Setareh Biotech LLC), was subsequently added to the medium for 90 min. Cells were fixed in 4% paraformaldehyde for 15 min, rinsed with PBS, and permeabilized with 0.1% Triton-X100 in PBS for 15 min. 0.5µg/ml of DAPI (Sigma-Aldrich) was used to counterstain DNA and coverslips were then mounted on slides.
   For Quantitative PCR of SASP and senescence-associated genes Total RNA from both asynchronous and mitotic cell populations was extracted using RNeasy^{®} Mini Kit (Qiagen). 1µg of total RNA was reverse-transcribed using the iScriptTM cDNA synthesis kit (Bio-Rad Laboratories). qPCR was performed using iTaq^{™} Universal SYBR^{®} Green Supermix in a CFX96/384 Touch^{™} Real-Time PCR Detection System and analyzed using the CFX Maestro Software (all from Bio-Rad Laboratories).
**Figure 3****: General chemical structure of UMK57:** 4-Pyrrolidin-1-Yl-5-P-Tolyl-Thieno[2,3-D] Pyrimidine.
**Figure 4****: Small molecule agonist of MCAK activity UMK57 rescues age-associated CIN and delays senescence.** (A) Mitotic duration of neonatal (N) and elderly (87y) human dermal fibroblasts (HDF) treated for 24 hrs with different concentrations of UMK57 (MCAK agonist). n≥58 cells were analyzed per condition. For all subsequent experiments UMK57 was used at 1µM for 24 hrs. (B) Representative images and (C) quantification of calcium-stable k-fiber intensity levels in metaphase, scored by immunofluorescence analysis of n≥34 tubulin-stained mitotic cells of elderly samples treated with DMSO (-) and UMK57 (+). Levels were normalized to neonatal DMSO-treated condition. Scale bar, 5µm. (D) Aneusomy index of chromosomes 7, 12 and 18 measured by interphase FISH analysis. (E) Percentage of cytochalasin D-induced binucleated (BN) cells with chromosomes 7, 12, and 18 mis-segregation (MS). (F) Percentage of micronuclei in n=cells scored when treated with DMSO or UMK57. (G) Percentage of n=cells staining positive for double immunostaining of Cdkn1a/p21 (cell cycle inhibitor) and 53BP1 (≥1 foci; DNA damage) senescence biomarkers. (H) Percentage of n=cells staining positive for SA-β-galactosidase (SA-β-gal) activity. All values are mean ± s.d. of at least two independent experiments. ns p>0.05, * p<0.05, ** p<0.01, *** p<0.001, **** p<0.0001 by two tailed (A,C) Mann-Whitney and (D-H) chi-square tests.
   1µM of UMK57 was used to enhance kinesin-13 activity during the time periods indicated for each experiment.
   FISH was used to score aneusomy indexes (Interphase FISH; Fig 4D, 5C) and chromosome mis-segregation (MS) rates. MS rates were scored by Cyto-D FISH (Fig 4E and 5D), or by FISH on STLC-washed-out fibroblasts (Fig 1G,H). For all experiments, fibroblasts were grown on Superfrost^{™} Plus microscope slides (Menzel, Thermo Fisher Scientific) placed in quadriperm dishes (Sarsted). Cells were fixed with ice-cold Carnoy fixative (methanol:glacial acetic acid, 3:1), following an initial 30 min hypotonic shock in 0.03M sodium citrate solution (Sigma-Aldrich). FISH was performed with Vysis centromeric probes CEP7 Spectrum Aqua, CEP12 Spectrum Green, and CEP18 Spectrum Orange (all from Abbott Laboratories) according to manufacturer's instructions. DNA was counterstained with 0.5µg/ml 4',6-Diamidino-2-Phenylindole (DAPI) and microscope slides were then mounted with coverslips in proper anti-fading medium (90% glycerol, 0.5% N-propyl gallate, 20mM Tris pH=8.0).
**Figure 5****: Long-term treatment with UMK57 does not lead to adaptive resistance in elderly cells.** (A) Mitotic duration of n=100 neonatal (N) and elderly (87y) human dermal fibroblasts (HDF) treated with DMSO (-) or UMK57 (+) for 96 hrs. (B) Relative calcium-stable k-fiber intensity levels scored by immunofluorescence analysis of n≥29 tubulin-stained metaphase cells of neonatal and elderly samples treated with DMSO and UMK57 for 96 hrs. Levels were normalized to neonatal DMSO-treated condition. (C) Aneusomy index of chromosomes 7, 12 and 18 measured by interphase FISH analysis of neonatal and elderly cells treated for 96 hrs. (D) Cytochalasin D-induced binucleated (BN) cells with mis-segregation (MS) of chromosomes 7, 12 and 18 in neonatal and elderly samples treated for 96 hrs. (E) Percentage of n=cells staining positive for double immunostaining of Cdkn1a/p21 (cell cycle inhibitor) and 53BP1 (≥1 foci; DNA damage) senescence biomarkers after 96hrs of treatment. (F) Percentage of n=cells staining positive for SA-β-gal activity when treated for 96 hrs with DMSO or UMK57. All values are mean ± s.d. of at least two independent experiments. ns p>0.05, * p<0.05, *** p<0.001, **** p<0.0001 by two tailed (A,B) Mann-Whitney and (C-F) chi-square tests.

### References

Abdul-Aziz AM, Sun Y, Hellmich C, Marlein CR, Mistry J, Forde E, Piddock RE, Shafat MS, Morfakis A, Mehta T, Di Palma F, Macaulay I, Ingham CJ, Haestier A, Collins A, Campisi J, Bowles KM, Rushworth SA (2019) Acute myeloid leukemia induces protumoral p16INK4a-driven senescence in the bone marrow microenvironment. Blood 133: 446-456
Baker DJ, Childs BG, Durik M, Wijers ME, Sieben CJ, Zhong J, Saltness RA, Jeganathan KB, Verzosa GC, Pezeshki A, Khazaie K, Miller JD, van Deursen JM (2016) Naturally occurring p16(Ink4a)-positive cells shorten healthy lifespan. Nature 530: 184-9 Baker DJ, Wijshake T, Tchkonia T, LeBrasseur NK, Childs BG, van de Sluis B, Kirkland JL, van Deursen JM (2011) Clearance of p16Ink4a-positive senescent cells delays ageing-associated disorders. Nature 479: 232-6
Bakhoum SF, Compton DA (2012) Kinetochores and disease: keeping microtubule dynamics in check! Curr Opin Cell Biol 24: 64-70 Bakhoum SF, Ngo B, Laughney AM, Cavallo JA, Murphy CJ, Ly P, Shah P, Sriram RK, Watkins TBK, Taunk NK, Duran M, Pauli C, Shaw C, Chadalavada K, Rajasekhar VK, Genovese G, Venkatesan S, Birkbak NJ, McGranahan N, Lundquist M et al. (2018) Chromosomal instability drives metastasis through a cytosolic DNA response. Nature 553: 467-472
Bakhoum SF, Thompson SL, Manning AL, Compton DA (2009) Genome stability is ensured by temporal control of kinetochore-microtubule dynamics. Nature cell biology 11: 27-35 Barroso-Vilares M, Logarinho E (2019) Chromosomal instability and pro-inflammatory response in aging. Mechanisms of ageing and development: 111118
Bussian TJ, Aziz A, Meyer CF, Swenson BL, van Deursen JM, Baker DJ (2018) Clearance of senescent glial cells prevents tau-dependent pathology and cognitive decline. Nature 562: 578-582
Childs BG, Baker DJ, Wijshake T, Conover CA, Campisi J, van Deursen JM (2016) Senescent intimal foam cells are deleterious at all stages of atherosclerosis. Science 354: 472-477
Childs BG, Durik M, Baker DJ, van Deursen JM (2015) Cellular senescence in aging and age-related disease: from mechanisms to therapy. Nature Medicine 21: 1424-35
Cimini D, Degrassi F (2005) Aneuploidy: a matter of bad connections. Trends Cell Biol 15: 442-51
Compton DA (2011) Mechanisms of aneuploidy. Current opinion in cell biology 23: 109-13
Ditchfield C, Johnson VL, Tighe A, Ellston R, Haworth C, Johnson T, Mortlock A, Keen N, Taylor SS (2003) Aurora B couples chromosome alignment with anaphase by targeting BubR1, Mad2, and Cenp-E to kinetochores. The Journal of Cell Biology 161: 267-280
Dou Z, Ghosh K, Vizioli MG, Zhu J, Sen P, Wangensteen KJ, Simithy J, Lan Y, Lin Y, Zhou Z, Capell BC, Xu C, Xu M, Kieckhaefer JE, Jiang T, Shoshkes-Carmel M, Tanim K, Barber GN, Seykora JT, Millar SE et al. (2017) Cytoplasmic chromatin triggers inflammation in senescence and cancer. Nature 550: 402-406
Drpic D, Almeida AC, Aguiar P, Renda F, Damas J, Lewin HA, Larkin DM, Khodjakov A, Maiato H (2018) Chromosome Segregation Is Biased by Kinetochore Size. Current Biology 28: 1344-1356.e5
Dudka D, Noatynska A, Smith CA, Liaudet N, McAinsh AD, Meraldi P (2018) Complete microtubule-kinetochore occupancy favours the segregation of merotelic attachments. Nature Communications 9: 2042
Ertych N, Stolz A, Stenzinger A, Weichert W, Kaulfuß S, Burfeind P, Aigner A, Wordeman L, Bastians H (2014) Increased microtubule assembly rates influence chromosomal instability in colorectal cancer cells. Nature cell biology 16: 779
Farr JN, Xu M, Weivoda MM, Monroe DG, Fraser DG, Onken JL, Negley BA, Sfeir JG, Ogrodnik MB, Hachfeld CM, LeBrasseur NK, Drake MT, Pignolo RJ, Pirtskhalava T, Tchkonia T, Oursler MJ, Kirkland JL, Khosla S (2017) Targeting cellular senescence prevents age-related bone loss in mice. Nature Medicine 23: 1072-1079
Fulop T, Larbi A, Dupuis G, Le Page A, Frost EH, Cohen AA, Witkowski JM, Franceschi C (2017) Immunosenescence and Inflamm-Aging As Two Sides of the Same Coin: Friends or Foes? Frontiers in immunology 8: 1960
Gao D, Wu J, Wu YT, Du F, Aroh C, Yan N, Sun L, Chen ZJ (2013) Cyclic GMP-AMP synthase is an innate immune sensor of HIV and other retroviruses. Science 341: 903-6
Gluck S, Guey B, Gulen MF, Wolter K, Kang TW, Schmacke NA, Bridgeman A, Rehwinkel J, Zender L, Ablasser A (2017) Innate immune sensing of cytosolic chromatin fragments through cGAS promotes senescence. Nature cell biology 19: 1061-1070
Harding SM, Benci JL, Irianto J, Discher DE, Minn AJ, Greenberg RA (2017) Mitotic progression following DNA damage enables pattern recognition within micronuclei. Nature 548: 466-470
He Q, Au B, Kulkarni M, Shen Y, Lim KJ, Maimaiti J, Wong CK, Luijten MNH, Chong HC, Lim EH, Rancati G, Sinha I, Fu Z, Wang X, Connolly JE, Crasta KC (2018) Chromosomal instability-induced senescence potentiates cell non-autonomous tumourigenic effects. Oncogenesis 7: 62
Hernandez-Segura A, de Jong TV, Melov S, Guryev V, Campisi J, Demaria M (2017) Unmasking Transcriptional Heterogeneity in Senescent Cells. Current Biology 27: 2652-2660 e4
Iourov IY, Vorsanova SG, Liehr T, Yurov YB (2009) Aneuploidy in the normal, Alzheimer's disease and ataxia-telangiectasia brain: differential expression and pathological meaning. Neurobiology of disease 34: 212-20
Jeon OH, Kim C, Laberge RM, Demaria M, Rathod S, Vasserot AP, Chung JW, Kim DH, Poon Y, David N, Baker DJ, van Deursen JM, Campisi J, Elisseeff JH (2017) Local clearance of senescent cells attenuates the development of post-traumatic osteoarthritis and creates a pro-regenerative environment. Nature Medicine 23: 775-781
Kollu S, Bakhoum SF, Compton DA (2009) Interplay of Microtubule Dynamics and Sliding during Bipolar Spindle Formation in Mammalian Cells. Current Biology 19: 2108-2113
Lampson MA, Renduchitala K, Khodjakov A, Kapoor TM (2004) Correcting improper chromosome-spindle attachments during cell division. Nature cell biology 6: 232-237
Lopez-Otin C, Blasco MA, Partridge L, Serrano M, Kroemer G (2013) The hallmarks of aging. Cell 153: 1194-217
Macedo JC, Vaz S, Bakker B, Ribeiro R, Bakker PL, Escandell JM, Ferreira MG, Medema R, Foijer F, Logarinho E (2018) FoxM1 repression during human aging leads to mitotic decline and aneuploidy-driven full senescence. Nature Communications 9: 2834 Macedo JC, Vaz S, Logarinho E (2017) Mitotic Dysfunction Associated with Aging Hallmarks. Advances in experimental medicine and biology 1002: 153-188
Mackenzie KJ, Carroll P, Martin CA, Murina O, Fluteau A, Simpson DJ, Olova N, Sutcliffe H, Rainger JK, Leitch A, Osborn RT, Wheeler AP, Nowotny M, Gilbert N, Chandra T, Reijns MAM, Jackson AP (2017) cGAS surveillance of micronuclei links genome instability to innate immunity. Nature 548: 461-465
Mahmoudi S, Xu L, Brunet A (2019) Turning back time with emerging rejuvenation strategies. Nature cell biology 21: 32-43
Manning AL, Ganem NJ, Bakhoum SF, Wagenbach M, Wordeman L, Compton DA (2007) The kinesin-13 proteins Kif2a, Kif2b, and Kif2c/MCAK have distinct roles during mitosis in human cells. Mol Biol Cell 18: 2970-9
Maresca TJ, Salmon ED (2009) Intrakinetochore stretch is associated with changes in kinetochore phosphorylation and spindle assembly checkpoint activity. The Journal of Cell Biology 184: 373-381
Melo Pereira S, Ribeiro R, Logarinho E (2019) Approaches towards Longevity: Reprogramming, Senolysis, and Improved Mitotic Competence as Anti-Aging Therapies. International journal of molecular sciences 20
Mosch B, Morawski M, Mittag A, Lenz D, Tarnok A, Arendt T (2007) Aneuploidy and DNA replication in the normal human brain and Alzheimer's disease. The Journal of Neuroscience 27: 6859-67 Mukherjee AB, Alejandro J, Payne S, Thomas S (1996) Age-related aneuploidy analysis of human blood cells in vivo by fluorescence in situ hybridization (FISH). Mechanisms of ageing and development 90: 145-56
Mukherjee AB, Thomas S (1997) A longitudinal study of human age-related chromosomal analysis in skin fibroblasts. Experimental Cell Research 235: 161-9
Nagaoka SI, Hassold TJ, Hunt PA (2012) Human aneuploidy: mechanisms and new insights into an age-old problem. Nature Reviews Genetics 13: 493-504
Orr B, Talje L, Liu Z, Kwok BH, Compton DA (2016) Adaptive Resistance to an Inhibitor of Chromosomal Instability in Human Cancer Cells. Cell Reports 17: 1755-1763
Roos CM, Zhang B, Palmer AK, Ogrodnik MB, Pirtskhalava T, Thalji NM, Hagler M, Jurk D, Smith LA, Casaclang-Verzosa G, Zhu Y, Schafer MJ, Tchkonia T, Kirkland JL, Miller JD (2016) Chronic senolytic treatment alleviates established vasomotor dysfunction in aged or atherosclerotic mice. Aging cell 15: 973-7
Santaguida S, Richardson A, Iyer DR, M'Saad O, Zasadil L, Knouse KA, Wong YL, Rhind N, Desai A, Amon A (2017) Chromosome Mis-segregation Generates Cell-Cycle-Arrested Cells with Complex Karyotypes that Are Eliminated by the Immune System. Developmental Cell 41: 638-651 e5
Stone JF, Sandberg AA (1995) Sex chromosome aneuploidy and aging. Mutation research 338: 107-13
Sun L, Wu J, Du F, Chen X, Chen ZJ (2013) Cyclic GMP-AMP synthase is a cytosolic DNA sensor that activates the type I interferon pathway. Science 339: 786-91
Swanson EC, Manning B, Zhang H, Lawrence JB (2013) Higher-order unfolding of satellite heterochromatin is a consistent and early event in cell senescence. The Journal of Cell Biology: jcb.201306073
van Deursen JM (2014) The role of senescent cells in ageing. Nature 509: 439-46
Yang H, Wang H, Ren J, Chen Q, Chen ZJ (2017) cGAS is essential for cellular senescence. Proceedings of the National Academy of Sciences 114: E4612-E4620
Zhai Y, Kronebusch PJ, Borisy GG (1995) Kinetochore microtubule dynamics and the metaphase-anaphase transition. Journal of Cell Biology 131: 721-34

## Claims

1. 4-Pyrrolidin-1-Yl-5-P-Tolyl-Thieno[2,3-D] Pyrimidine for use in the treatment of aging-associated diseases selected from the group consisting of: atherosclerosis, cardiovascular disease, arthritis, cataracts, osteoporosis, type 2 diabetes, hypertension and Alzheimer's disease.

2. 4-Pyrrolidin-1-Yl-5-P-Tolyl-Thieno[2,3-D] Pyrimidine for use according to claim 1, wherein treatment comprises administering said substance systemically, orally or by local injection.

3. A pharmaceutical composition comprising 4-Pyrrolidin-1-Yl-5-P-Tolyl-Thieno[2,3-D] Pyrimidine as active ingredient for use in the treatment of aging-associated diseases selected from the group consisting of: atherosclerosis, cardiovascular disease, arthritis, cataracts, osteoporosis, type 2 diabetes, hypertension and Alzheimer's disease.

4. A pharmaceutical composition for use according to claim 3 wherein treatment comprises administering said pharmaceutical composition systemically, orally or by local injection.

5. 4-Pyrrolidin-1-Yl-5-P-Tolyl-Thieno[2,3-D]Pyrimidine for use in the treatment of premature aging diseases selected from the group consisting of: Bloom Syndrome; Cockayne Syndrome Type I or Type III; Hutchinson-Gilford Progeria Syndrome; Mandibuloacral Dysplasia with Type A Lipodystrophy; Progeria of Adult Onset; Progeroid Syndrome, Neonatal Rothmund-Thomson Syndrome; Seip Syndrome and Werner Syndrome.

6. 4-Pyrrolidin-1-Yl-5-P-Tolyl-Thieno[2,3-D] Pyrimidine for use according to claim 5, wherein treatment comprises administering said substance systemically, orally or by local injection.

7. A pharmaceutical composition comprising 4-Pyrrolidin-1-Yl-5-P-Tolyl-Thieno[2,3-D] Pyrimidine as active ingredient for use in the treatment of premature aging diseases selected from the group consisting of: Bloom Syndrome; Cockayne Syndrome Type I or Type III; Hutchinson-Gilford Progeria Syndrome; Mandibuloacral Dysplasia with Type A Lipodystrophy; Progeria of Adult Onset; Progeroid Syndrome, Neonatal Rothmund-Thomson Syndrome; Seip Syndrome and Werner Syndrome.

8. A pharmaceutical composition for use according to claim 7 wherein treatment comprises administering said pharmaceutical composition systemically, orally or by local injection.

## Patentansprüche

1. 4-Pyrrolidin-1-yl-5-p-tolyl-thieno[2,3-D]pyrimidin zur Behandlung altersbedingter Erkrankungen, ausgewählt aus der Gruppe bestehend aus: Arteriosklerose, Herz-Kreislauf-Erkrankungen, Arthritis, Katarakt, Osteoporose, Typ-2-Diabetes, Hypertonie und Alzheimer-Krankheit.

2. 4-Pyrrolidin-1-yl-5-p-tolyl-thieno[2,3-D]pyrimidin zur Verwendung nach Anspruch 1, wobei die Behandlung die systemische, orale Verabreichung oder lokale Injektionsverabreichung des Wirkstoffs umfasst.

3. Eine pharmazeutische Zusammensetzung, die 4-Pyrrolidin-1-yl-5-p-tolyl-thieno[2,3-D]pyrimidin als Wirkstoff enthält, zur Behandlung altersbedingter Erkrankungen, ausgewählt aus der Gruppe bestehend aus: Arteriosklerose, Herz-Kreislauf-Erkrankungen, Arthritis, Katarakt, Osteoporose, Typ-2-Diabetes, Hypertonie und Alzheimer-Krankheit.

4. Eine pharmazeutische Zusammensetzung zur Verwendung nach Anspruch 3, wobei die Behandlung die systemische, orale Verabreichung oder lokale Injektionsverabreichung der pharmazeutischen Zusammensetzung umfasst.

5. 4-Pyrrolidin-1-yl-5-p-tolyl-thieno[2,3-D]pyrimidin zur Behandlung von vorzeitigen Alterungserkrankungen, ausgewählt aus der Gruppe bestehend aus: Bloom-Syndrom; Cockayne-Syndrom Typ I oder Typ III; Hutchinson-Gilford-Progerie-Syndrom; Mandibuloakrale Dysplasie mit Lipodystrophie Typ A; Progerie im Erwachsenenalter; Progeroides Syndrom, neonatales Rothmund-Thomson-Syndrom; Seip-Syndrom und Werner-Syndrom.

6. 4-Pyrrolidin-1-yl-5-p-tolyl-thieno[2,3-D]pyrimidin zur Verwendung nach Anspruch 5, wobei die Behandlung die systemische, orale Verabreichung oder lokale Injektionsverabreichung des Wirkstoffs umfasst.

7. Eine pharmazeutische Zusammensetzung, die 4-Pyrrolidin-1-yl-5-p-tolyl-thieno[2,3-D]pyrimidin als Wirkstoff enthält, zur Behandlung von vorzeitigen Alterungserkrankungen, ausgewählt aus der Gruppe bestehend aus: Bloom-Syndrom; Cockayne-Syndrom Typ I oder Typ III; Hutchinson-Gilford-Progerie-Syndrom; Mandibuloakrale Dysplasie mit Lipodystrophie Typ A; Progerie im Erwachsenenalter; Progeroides Syndrom, neonatales Rothmund-Thomson-Syndrom; Seip-Syndrom und Werner-Syndrom.

8. Eine pharmazeutische Zusammensetzung zur Verwendung nach Anspruch 7, wobei die Behandlung die systemische, orale Verabreichung oder lokale Injektionsverabreichung der pharmazeutischen Zusammensetzung umfasst.

## Revendications

1. 4-Pyrrolidin-1-yl-5-p-tolyl-thiéno[2,3-d]pyrimidine pour utilisation dans le traitement de maladies liées au vieillissement, sélectionné parmi le groupe constitué de: l'athérosclérose, les maladies cardiovasculaires, l'arthrite, la cataracte, l'ostéoporose, le diabète de type 2, l'hypertension et la maladie d'Alzheimer.

2. 4-Pyrrolidin-1-yl-5-p-tolyl-thiéno[2,3-d]pyrimidine, utilisée selon la revendication 1, le traitement comprenant l'administration de ladite substance par voie systémique, orale ou par injection locale.

3. Composition pharmaceutique comprenant de la 4-pyrrolidin-1-yl-5-p-tolyl-thiéno[2,3-d]pyrimidine comme principe actif, destinée au traitement de maladies liées au vieillissement, sélectionné parmi le groupe constitué de: l'athérosclérose, les maladies cardiovasculaires, l'arthrite, la cataracte, l'ostéoporose, le diabète de type 2, l'hypertension et la maladie d'Alzheimer.

4. Composition pharmaceutique selon la revendication 3, le traitement comprenant l'administration de ladite composition par voie systémique, orale ou par injection locale.

5. 4-Pyrrolidin-1-yl-5-p-tolyl-thiéno[2,3-d]pyrimidine destinée au traitement de maladies du vieillissement prématuré, sélectionné parmi le groupe constitué de: le syndrome de Bloom; le syndrome de Cockayne de type I ou de type III; le syndrome de Hutchinson-Gilford Progéria; la dysplasie mandibulo-acrale avec lipodystrophie de type A; la progéria de l'adulte; le Syndrome progéroïde, le syndrome néonatal de Rothmund-Thomson; le syndrome de Seip et le syndrome de Werner.

6. 4-Pyrrolidin-1-yl-5-p-tolyl-thiéno[2,3-d]pyrimidine, utilisée selon la revendication 5, le traitement comprenant l'administration de ladite substance par voie systémique, orale ou par injection locale.

7. Composition pharmaceutique comprenant de la 4-Pyrrolidin-1-yl-5-p-tolyl-thiéno[2,3-d]pyrimidine comme principe actif, destinée au traitement des maladies de vieillissement prématuré sélectionné parmi le groupe constitué de: syndrome de Bloom; syndrome de Cockayne de type I ou III; syndrome de Hutchinson-Gilford Progéria; dysplasie mandibulo-acrale avec lipodystrophie de type A; progéria de l'adulte; syndrome progéroïde, syndrome néonatal de Rothmund-Thomson; syndrome de Seip et syndrome de Werner.

8. Une composition pharmaceutique à utiliser selon la revendication 7, le traitement comprenant l'administration de ladite composition pharmaceutique par voie systémique, orale ou par injection locale.
